# EUROPEAN PATENT APPLICATION

(11) **EP 1 360 898 A1**
(43) Date of publication of application: **12.11.2003**
(21) Application number: 01271144.6
(22) Date of filing: 19.12.2001
(51) Int. Cl.: A01K 67/027, C12N 15/52, C12N 15/85, C12N 15/86, C12N 5/10, A61K 48/00

(54) **JOINT UTILIZATION OF THE INSULIN GENE AND THE GLUCOKINASE GENE IN THE DEVELOPMENT OF THERAPEUTIC APPROACHES FOR DIABETES MELLITUS**

(30) Priority: 20.12.2000 ES 200003056
(71) Applicant: UNIVERSITAT AUTONOMA DE BARCELONA, 08193 Bellaterra (ES)
(72) Inventor: BOSCH TUBERT, Fatima, E-08290 Cerdanyola del Valles (ES); RIU PASTOR, Efren, E-08940 Cornella (ES); OTAEGUI GOYA, Pedro, Jose, E- Sant Cugat del valles (ES); FERRE MASFERRER, MA DEL TURA, E-08015 Barcelona (ES); MAS MONTEYS, Alejandro, E-08025 Barcelona (ES)
(74) Representative: Ponti Sales, Adelaida
(86) International application number: ES0100493
(87) International publication number: WO02049423

(57) **Abstract**

This invention relates to a double transgenic animal that simultaneously expresses the gene or the cDNA (complementary DNA) of insulin and the gene or the cDNA (complementary DNA) of glucokinase directed by a promoter or fusion of promoters which permit insulin and glucokinase to be expressed in muscle and its use in the development of therapeutic approximations for diabetes mellitus.
This invention also relates to a vector or vectors of expression which permits the expression of said chimeric genes jointly in muscle cells. Said vectors can be a plasmid, a viral vector or a non-viral vector.

## Description

### FIELD OF THE INVENTION

This invention relates to a double transgenic animal that simultaneously expresses the gene or the cDNA (complementary DNA) of insulin and the gene or the cDNA (complementary DNA) of glucokinase directed by a promoter or fusion of promoters which permit insulin and glucokinase to be expressed in muscle and its use in the development of therapeutic approximations for diabetes mellitus.

### BACKGROUND OF THE INVENTION

Diabetes mellitus is the most common metabolic illness. It includes a wide variety of syndromes with different etiologies which together affect between 2% and 7% of the world population. Between 5 and 10% of patients can be grouped under the category of insulin-dependent diabetes mellitus or type 1 diabetes, which generally manifests itself before the age of 40 years, frequently during adolescence, and is the result of autoimmune destruction of the β cells of the islets of Langerhans in the pancreas. Much more common is type 2 diabetes or non-insulin-dependent diabetes mellitus, which manifests itself in adult individuals and which, at least in the initial stages, is not characterised by an insulin deficiency but rather the inability of the hormone to act efficiently in target tissues such as muscle, liver or adipose tissue [The Expert Committee on the Diagnosis and Classification of Diabetes Mellitus (1997). *Diabetes Care* 20, 1183-1197; McGarry, J.D. (1992). *Science* 258, 766-770; De Fronzo, R.A. (1997). *Diab. Rev. 5,* 177-269]. The incidence of type 2 diabetes in our country is around 10% of the population aged over 30 years, although many sufferers are not detected until very advanced stages of the illness. Approximately 45% of the men and 70% of the women with type 2 diabetes are obese.

As mentioned above, insulin-dependent diabetes mellitus (or type 1 diabetes) is the result of autoimmune destruction of the pancreatic β cells, which leads to insulin deficiency, hyperglycaemia and the development of microvascular, macrovascular and neurological complications. The risk of suffering these complications increases in function of the degree of hyperglycaemia. Patients with type 1 diabetes depend dramatically on administration of the hormone. Interruption or lack of treatment with insulin leads, firstly, to hyperglycaemia, later to coma and finally to death of the ill person if the hormone is not injected. Although therapy with insulin allows most patients to lead an active life, this replacement is imperfect and has a considerable effect on their life-style. Intensive therapy with insulin can delay and slow down the appearance and progression of microvascular complications. This type of treatment cannot be carried out in all diabetic patients, however, and is not advisable in children or the elderly. Moreover, patients undergoing this intensive treatment with insulin present a high risk of hypoglycaemia.

All the forms of diabetes are characterised by the presence of hyperglycaemia, which has been postulated as the main factor responsible for the development of microvascular pathology in the retina and in the kidney and responsible for the neurological complications. As a consequence of this microvascular pathology, diabetes mellitus is at present the main cause of blindness in adults and is responsible for one third of cases of chronic kidney failure. Patients with type 2 diabetes also present an increased risk of developing premature atherosclerosis and increased mortality from myocardial infarction, cerebrovascular disease and peripheral vascular disease [Pickup, J.C. and Williams, G. (1994). *Chronic Complications of Diabetes,* Blackwell Scientific Publications, Oxford, UK]. The trial carried out by the *Diabetes Control and Complications Trial* (DCCT) has shown that intensive therapy with insulin (three or more injections daily) can delay the onset and progression of retinotherapy, nephropathy and neuropathy in type 1 diabetes patients [The Diabetes Control and Complications Trial Research Group (1993). *N. Engl. J. Med.* 329, 977-986; American Diabetes Association (1993). *Diabetes* 42, 1555-1558]. An improvement in the control of glycaemia could also reduce microvascular complications in type 2 diabetes patients. However, the use of this therapy in these patients could exacerbate the macrovascular complications, which are the main cause of mortality in this illness. Hyperinsulinaemia and resistance to insulin, very common in type 2 diabetes patients, are associated with increased risk of suffering hypertension, heart disease and myocardial infarction, which leads to consideration of the possibility that insulin of itself has atherogenic action. Furthermore, this type of treatment cannot be carried out in all type 1 diabetes patients, and especially not in the very young and the elderly. Furthermore, patients undergoing treatment with intensive insulin therapy present a very high risk of developing hypoglycaemia.

At present, most patients are treated with subcutaneous injections of recombinant human insulin preparations that aim to limitate the physiological profiles of the hormone (low basal levels upon which are superimposed postprandial peaks of insulin secretion). The insulin administered in solution is absorbed rapidly and is fast in its action, while suspensions of insulin particles of different sizes provide intermediate and long action. However, the mixture of soluble with slow insulin reduces the availability of the fast-acting components [Heine et al. (1985) *Bio. Med.* J. 290, 204-205]. One of the main deficiencies of the delayed-action insulins is their variable absorption from the subcutaneous tissue [Binder et al. (1984). *Diabetes Care* 7, 188-199]. Moreover, the delayed-action preparations are not generally capable of producing suitable basal levels of insulin, and often result in the appearance of both hyperglycaemia and hypoglycaemia.

Owing to the fact that replacement therapy with insulin is not perfect, attempts have been made to carry out pancreas transplants and also transplants of islets [Remuzzi et al. (1994); *Lancet* 343, 27-31]. These approximations aim to remove the need for daily injections of insulin, but require chronic immunosuppression and have not provided very successful results. Moreover, the number of donors is very limited and, therefore, the treatment of a large number of patients would not appear to be very realistic.

The approximation of this invention is based on genetically manipulating muscle cells so that they produce, simultaneously, a type of proinsulin which can be processed completely by the endoproteases present in the constitutive secretion pathway in the muscle, so as to provide biologically active human insulin, and the glucose phosphorylising enzyme glucokinase which permits an increase in uptake of glucose by the skeletal muscle and leads to a reduction of diabetic hyperglycaemia.

### DESCRIPTION OF THE INVENTION

This invention relates to a non-human transgenic animal which simultaneously expresses the gene or the cDNA of insulin or derivatives and the gene or the cDNA of glucokinase directed by a promoter or fusion of promoters that permit expression of these genes in muscle cells during the diabetic process.

This invention also relates to a vector or vectors of expression which permits combined expression of the chimeric genes described above in muscle cells. Said vectors can be a plasmid, a viral vector or a non-viral vector.

In the case of a viral vector, this can be a retroviral vector, an adenoviral vector, an adeno-associated vector, a Sindbis viral vector, lentiviral vector or a vector derived from the herpes virus.

The animals of the invention express the chimeric gene which contains the cDNA of mutated human insulin (Ins*) under control of the promoter of the myosin light chain (MLC) and the cDNA of glucokinase of rat under the control of the same MLC promoter. The MLC promoter confers constitutive expression in skeletal muscle and in myotubes under culture [Donoghue et al. (1988) *Genes Dev.* 2, 1779-1790; Rosenthal et al. (1989) *Proc. Natl. Acad. Sci. USA* 86, 7780-7784]. The cDNA of the human insulin was obtained by digestion of the plasmid pP2.41nsm [Gros et al. (1997) Hum. *Gene Ther.* 8, 2249-2259], and it was then inserted into a plasmid that contained the promoter and regulating sequence of the gene of the MLC.

Also considered an object of this invention is a muscle cell which expresses at the same time the chimeric genes described in the invention. The chimeric genes are introduced into the cell by means of the vector described, for use thereof in the development of therapeutic approximations for diabetes mellitus.

Another objective of this invention is a device which contains the muscle cells described above, which are packed.

An object of this invention is combined utilisation of the chimeric genes described above for the development of therapeutic approximations for diabetes mellitus, together with use of the vector or vectors of expression of the invention for utilisation thereof in the development of therapeutic approximations for diabetes mellitus.

The double transgenic animals of this invention were obtained by crossing of transgenic animals which express a chimeric gene that includes the gene or cDNA (complementary DNA) of human insulin directed by a promoter or fusion of promoters with transgenic animals which express the gene or cDNA (complementary DNA) of glucokinase of rat directed by a promoter or fusion of promoters. In particular, said transgenic animal is a mouse.

In order to study the effects of the combined production of insulin and of glucokinase by the skeletal muscle double transgenic mice were obtained which simultaneously expressed the chimeric gene MLC/Ins*, which contained the cDNA of mutated human insulin (Ins*) under the control of the promoter of the myosin light chain (MLC), and of the chimeric gene MLC/GK, which contained the cDNA of the glucokinase of rat (GK) under the control of the same MLC promoter.

Following killing of the animals, expression of the chimeric genes was analysed. This determined the presence of mRNA of insulin and of glucokinase in the skeletal muscles of the double transgenic mice. The results indicated that the double transgenic mice presented both bands corresponding to the mRNA of insulin and of glucokinase and that the expression of both chimeric genes in constitutive manner by the skeletal muscle was compatible with normal survival of the animal. At three months of age, the double transgenic mice presented glycaemia levels similar to those of the control mice.

A test of tolerance to glucose was carried out in order to study whether the simultaneous expression of insulin and glucokinase in the skeletal muscle could affect the metabolism of glucose *in vivo.* The double transgenic mice presented lower levels of glucose than the control mice throughout the entire test, which indicated that the combined expression of insulin and glucokinase increased uptake of glucose.

Subsequently, the behaviour of said double transgenic mice under conditions of hyperglycaemia was analysed. To that end, experimental diabetes was induced in both the double transgenic mice and the control mice by means of injecting intraperitoneally for five consecutive days the toxic streptozocin (Stz). Analysis was then carried out of expression of the chimeric genes in the skeletal muscle of the diabetic double transgenic animals, and it was found that these animals maintained expression of insulin and glucokinase under these conditions.

At three weeks from the start of the treatment with Stz, the control mice showed a considerable rise in glucose concentration, while the double transgenic mice only showed a slight rise in the concentration of circulating glucose. Therefore, the simultaneous expression of insulin and glucokinase in the skeletal muscle was capable of counteracting the diabetic hyperglycaemia.

A test of tolerance to insulin and a test of tolerance to glucose were also carried out on the diabetic animals. In the former, the double transgenic mice showed a more rapid and more significant response than the controls treated with Stz, a response similar to that of non-treated control mice, while in the latter the double transgenic mice treated with Stz showed throughout the test glucose levels lower than those of the controls treated with Stz, indicating that these double transgenic animals had greater glucose uptake capacity.

The intramuscular concentration of glucose-6-phosphate, glucogen and lactate were also determined. Production of insulin and glucokinase by the skeletal muscle after treatment with Stz in the double transgenic mice enhanced greater utilisation and greater storage of the intramuscular glucose in relation to the diabetic control mice.

In parallel to this, the levels of various metabolites related with the hepatic metabolism of glucose in the mice treated with Stz were determined. The control mice showed a marked reduction in the concentrations of glucose-6-phosphate and of glucogen. The double transgenic mice, on the other hand, showed a normalisation of these parameters.

### EXAMPLES

The examples which follow are by way of non-restrictive illustration of the invention.

### Example I. Transgenic mouse which expresses the cDNA of mutated human insulin (Ins*) and the cDNA of glucokinase of rat in skeletal muscle

### I.1 Obtaining the transgenic mouse

The transgenic mouse was obtained from the crossing of transgenic mice which express the chimeric gene that contains the cDNA of mutated human insulin (Ins*) under control of the promoter of the myosin light chain (MLC) with transgenic mice which express the cDNA of glucokinase of rat under control of the same MLC promoter.

Three weeks after birth the presence of the two transgenes in the offspring was analysed by means of Southern transfer and hybridisation analysis (*Southern blot)* of 10 µg of DNA taken from the tail of each one of the animals. The DNA samples were digested with the restriction enzymes *Hind* III and *Eco* RI. These digestions resulted in a band of 1.9 Kb., corresponding to the MLC-mutated insulin promoter chimeric gene and a band of 2.3 Kb. corresponding to the MLC-glucokinase promoter chimeric gene. The double transgenic animals were thus those that presented both characteristic fragments.

### I.2 Analysis of expression of the transgene

Expression of the chimeric genes in the muscle of the transgenic mice was found by means of analysis of the total RNA extracted from the skeletal muscle of the control and double transgenic animals. The total RNA was obtained by using the isothiocyanate method [Chirwin et al. (1979) *Biochemistry* 18, 5294-5299], which was followed by separation of RNA samples (30 µg) in an electrophoresis gel of 1% agarose containing formaldehyde 2.2 M and transfered *(Northern blot)* to a membrane for hybridisation thereof, using as probes the cDNA of the mutated human insulin and the cDNA of the rat glucokinase, both obtained by means of digestion with EcoRI. To that end, the probes were marked with [α⁻³²P] dCTP by means of the oligopriming method in accordance with the manufacturer's instructions. The activity of the probes was approximately 10⁹ cpm/µg DNA. The transfer membranes were placed in contact with Kodak XAR-5 films. The presence of hybridisation corresponding to the RNA of the insulin and the RNA of the glucokinase in the RNA samples obtained from the skeletal muscles of the double transgenic mice indicates expression of both chimeric genes in said tissue, while neither of the two bands was observed in the samples taken from the muscles of the control mice.

### I.3 Test for tolerance to glucose

The glucose tolerance test is one of the tests used in hospitals for diagnosing resistance to insulin. In order to determine whether the constitutive expression of insulin and of glucokinase at muscular level could alter the metabolism of glucose *in vivo,* a test for glucose tolerance was carried out under fasting conditions in control mice and double transgenic mice aged 3 to 4 months. No differences were observed in the basal level of glycaemia of the two groups. In the course of the test it was observed that the glycaemia levels of the double transgenic mice were lower than those of the control mice group, indicating that the production of insulin and of glucokinase by the skeletal muscle was increasing glucose uptake, thus improving the response to changes in the concentration of glucose.

### I.4 Obtaining diabetic mice

In order to analyse whether the simultaneous expression of insulin and glucokinase in the muscle of the double transgenic mice was capable of counteracting the hyperglycaemia of the diabetic process, experimental diabetes was induced in the control and double transgenic mice by intraperitoneal injection of streptozocin (Stz) dissolved in a 10 mM sodium citrate buffer solution with 0.9% NaCl pH 4.5, for five consecutive days, at a dosage of 45 mg Stz/kg live weight. Diabetes was confirmed by measuring blood glucose levels.

### I.5 Analysis of the glucose levels

The concentration of glucose was determined by means of the Glucometer Elite™ system from Bayer.

The blood glucose levels of the control and double transgenic mice treated with Stz and of the untreated control mice were analysed. At three weeks from the start of treatment with Stz the blood glucose levels of the control mice had increased fourfold, and they presented marked hyperglycaemia (578±15 mg/dl), while the double transgenic mice presented circulating glucose levels close to normoglycaemia, with an increase of only 1.5 times observed when compared with the untreated control mice (231±35 mg/dl in front of 150±5 mg/dl). Over the course of the three months' duration of the experiment it was observed that in the double transgenic mice the blood glucose levels remained constant, without tendency to increase, whereas in the control mice the blood glucose levels continued to increase, so that at 6 weeks from the start of the treatment with Stz the glucose levels exceeded the limit of detection of the analyser used (>600 mg/dl). This indicated that the simultaneous expression of insulin and of glucokinase in the double transgenic mice was leading to increased uptake of glucose by the skeletal muscle, thus preventing the development of hyperglycaemia.

### I.6 Test of tolerance to insulin in mice treated with Stz

In order to analyse whether the double transgenic animals treated with Stz could be more sensitive to insulin than the control animals treated with Stz, an insulin tolerance test was carried out 21 days after administration of the drug. 0.75 U of soluble insulin for each kg of weight was injected into healthy control mice and control and double transgenic mice treated with Stz and the existence of glycaemia was determined at regular intervals of time.

The hyperglycaemic response to the administration of the hormone observed in the double transgenic mice was more rapid and more significant than that of the treated control mice and similar to that of the control mice not treated with Stz. At 15 minutes from administration of insulin the basal glycaemia of the double transgenic mice had already reduced by 20%, whereas in the diabetic control mice no significant reduction was observed in the glycaemia values until 30 minutes after administration of the hormone, when a reduction of only 10% was observed with respect to the basal level. This effect was greater at 60 minutes, since the double transgenic mice showed a reduction of nearly 50% (from 232±43 mg/dl to 99±21 mg/dl), achieving normoglycaemic values, while the control mice continued to be markedly hyperglycaemic, with a reduction of 20% (from 593±7 mg/dl to 521±28 mg/dl).

### I.7 Test of tolerance to glucose in mice treated with Stz

A test of tolerance to glucose was carried out under fasting conditions in control and double transgenic mice three months after administration of Stz. 1 mg of glucose was injected per g of live weight.

Significant differences in the basal levels of glycaemia were observed. The control mice treated with Stz presented fasting glycaemia levels characteristic of a diabetic condition (326±14 mg/dl), while in the double transgenic mice treated with Stz the blood glucose levels did not differ from those observed in the untreated control mice (115±4 mg/dl vs 102±12 mg/dl). At 15 minutes from administration of glucose an increase in glycaemia was observed in all groups. The increases observed in the glucose levels of the control and double transgenic mice treated with Stz were, however, higher than those reached in the untreated control mice. Furthermore, during the carrying out of the test it was observed that both the untreated control mice and the double transgenic mice treated with Stz recovered the basal rates of glycaemia after 3 hours. On the other hand, the control mice treated with Stz did not regain the basal levels.

### I.8 Serum parameters in mice treated with Stz

The insulin was determined by means of radioimmunoassay (RIA) with the INSULIN-CT commercial kit from CIS Biointern ational, France.

Three months after administration of Stz the serum levels of insulin, glucose, triglycerides and protein were analysed in the untreated control mice and in the control and double transgenic mice treated with Stz. In the control mice treated with Stz a reduction of insulinaemia of approximately 50% was observed in relation to the untreated control mice, while in the double transgenic mice treated with Stz insulinaemia remained normal. Owing to the reduction of blood insulin levels, the control mice treated with Stz presented marked hyperglycaemia, together with altered levels of triglycerides and proteins (Table 1). Despite the fact that they presented normal insulinaemia, in the double transgenic mice it was observed that blood glucose levels were slightly increased (Table 1), possibly due to part of the insulin secreted by the skeletal muscle not being mature insulin but rather proinsulin. Nevertheless, the levels of triglycerides and proteins observed in the double transgenic mice were normalised (Table 1).

**Table 1:**

| Determination of serum parameters | | | |
|---|---|---|---|
| | Insulin (µU/ml) | Glucose (mg/dl) | Triglycerides (mg/dl) |
| Control | 35±3 | 138±9 | 98±9 |
| Control Stz | 19±1 | >600 | 179±22 |
| Transg.Stz | 34±1 | 244±41 | 119±17 |

### I.9 Expression of the chimeric genes during the diabetic state

In order to analyse expression of the chimeric genes MLC/Insulin and MLC/Glucokinase in skeletal muscle of animals treated with Stz, 15 µg of total RNA from muscle were used from untreated control mice and double transgenic mice at 3 months from treatment with Stz. Northern blot analysis showed that the skeletal muscle of the double transgenic mice still maintained expression of the chimeric genes following administration of Stz.

### I.10 Analysis of muscular parameters

Samples were taken of skeletal muscle of control and double transgenic mice, both Stz treated and untreated. The intracellular levels of glucose-6-phosphate were determined by an electrophotometric method [Michal, G. (1981) *Methods of Enzymatic Analysis,* vol. VI, 185-190], using the Cobas Bio (Roche) autoanalyser. The intracellular levels of glucogen were also determined by the α-amyloglucosidase method [Keppler, D. and Decker, K. (1981) *Methods of Enzymatic Analysis,* vol. VI, 11-18]. The glucose released by the enzyme was determined spectrophotometrically with the Cubas Bio autoanalyser, using the Gluco-Quant commercial equipment from Boehringer Mannheim. The levels of lactate were likewise determined using the lactate dehydrogenase method [Boehringer Mannheim].

As Table 2 shows, the double transgenic mice treated with Stz presented levels of the various metabolites equal to or greater than those observed in the untreated control mice, while in the control mice treated with Stz the levels of metabolites were altered, which indicates that expression of insulin and glucokinase by the skeletal muscle would aid the re-establishment of the metabolic pathways involved in the utilisation and accumulation of glucose.

**Table 2:**

| Determination of metabolites in skeletal muscle. | | | |
|---|---|---|---|
| | Glucose-6-phosphate (nmol/g tissue) | Lactate (mg/g tissue) | Glucogen (mg/g tissue) |
| Control | 601±49 | 18.9±0.8 | 2.3±0.2 |
| Control Stz | 409±47 | 17.1±1.67 | 1.4±0.3 |
| Transg. Stz | 665+16 | 19.9±1.1 | 2.1±0.2 |

### I.11 Analysis of the hepatic parameters

In order to determine if the expression of insulin by skeletal muscle could have an effect on other tissues of the organism, an analysis was made of utilisation and accumulation of glucose in the liver of mice treated with Stz. Samples of hepatic tissue were obtained from Stz-treated and untreated control mice and from double transgenic mice treated with Stz. The concentrations of glucose-6-phosphate and of glucogen were determined following the same methods as described above.

As Table 3 shows, the double transgenic mice treated with Stz showed normalised levels of metabolites related with uptake and utilisation of glucose, while in the control mice treated with Stz said metabolites were altered. This indicated that expression of insulin by the skeletal muscle favoured re-establishment of the metabolic pathways involved in the utilisation and accumulation of hepatic glucose.

**Table 3:**

| Determination of hepatic metabolites | | |
|---|---|---|
| | Glucose-6-phosphate (nmol/g tissue) | Glucogen (mg/g tissue) |
| Control | 1300±238 | 74±3 |
| Control Stz | 870±115 | 51±7 |
| Transg. Stz | 1200±87 | 70±6 |

## Claims

1. Non-human transgenic animal that simultaneously expresses the gene or the cDNA of insulin or derivatives directed by a promoter or fusion of promoters which permit insulin or derivatives of insulin to be expressed in muscle cells and the gene or the cDNA of glucokinase directed by a promoter or fusion of promoters which permit glucokinase to be expressed in muscle cells.

2. Vector or vectors of expression which permit the expression of the chimeric genes jointly as claimed in Claim 1 in muscle cells.

3. Vector or vectors of expression as claimed in Claim 2, said vectors being a plasmid.

4. Vector or vectors of expression as claimed in Claim 2, said vectors being a viral vector.

5. Vector or vectors of expression as claimed in Claim 2, said vectors being a non-viral vector.

6. Viral vector as claimed in Claim 4, this vector being a retroviral vector, an adenoviral vector, an adeno-associated viral vector, a Sindbis viral vector, a lentiviral vector or a vector derived from the herpes virus.

7. Muscle cell which expresses jointly the chimeric genes as claimed in Claim 1.

8. Muscle cell as claimed in Claim 7, into which the chimeric genes have been introduced by means of a vector as claimed in any of Claims 2 to 6, for use thereof in the development of therapeutic approximations for diabetes mellitus.

9. Device which contains muscle cells as claimed in either of Claims 7 and 8, packed.

10. Use of the chimeric genes as claimed in Claim 1 for utilisation thereof in the development of therapeutic approximations for diabetes mellitus.

11. Use of a vector of expression as claimed in any of Claims 2 to 6 for utilisation thereof in the development of therapeutic approximations for diabetes mellitus.

12. Use of a device as claimed in Claim 9 for the development of therapeutic approximations for diabetes mellitus.
